(19) Europäisches Patentamt
European Patent Office
Office européen des brevets

(11) Veröffentlichungsnummer: **0 280 187 B1**

(12) # EUROPÄISCHE PATENTSCHRIFT

(45) Veröffentlichungstag der Patentschrift: **02.01.92**

(51) Int. Cl.⁵: **A61K 7/13**, C07C 229/52, C07C 303/00, C07C 307/00, C07C 309/00, C07C 311/00

(21) Anmeldenummer: **88102336.0**

(22) Anmeldetag: **18.02.88**

(54) **Haarfärbemittel mit direktziehenden Nitrodiphenylamin-Derivaten.**

(30) Priorität: **26.02.87 DE 3706225**

(43) Veröffentlichungstag der Anmeldung:
**31.08.88 Patentblatt 88/35**

(45) Bekanntmachung des Hinweises auf die Patenterteilung:
**02.01.92 Patentblatt 92/01**

(84) Benannte Vertragsstaaten:
**AT BE CH DE ES FR GB GR IT LI LU NL SE**

(56) Entgegenhaltungen:
**EP-A- 0 010 244**
**EP-A- 0 195 361**
**EP-A- 0 220 613**
**GB-A- 1 517 105**
**US-A- 4 494 953**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Rose, David, Dr.**
**Holbeinweg 7**
**W-4010 Hilden(DE)**
Erfinder: **Lieske, Edgar**
**Hunsrückenstrasse 40**
**W-4000 Düsseldorf(DE)**
Erfinder: **Höffkes, Horst, Dr.**
**Carlo-Schmid-Strasse 113**
**W-4000 Düsseldorf-Hellerhof(DE)**
Erfinder: **Maak, Nobert, Dr.**
**Im Jagdfeld 41 a**
**W-4040 Neuss(DE)**

**EP 0 280 187 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Haarfärbemittel mit einem Gehalt an direktziehenden Haarfarbstoffen. Solche Haarfärbemittel enthalten direktziehende Haarfarbstoffe in einem kosmetischen Träger. Oft enthalten solche Haarfärbemittel zur Erzielung bestimmter Nuancen zusätzlich Oxidationsfarbstoffvorprodukte. Als kosmetische Träger für die direktziehenden Haarfarbstoffe und gegebenenfalls Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und ermatologischer Hinsicht unbedenklich sein.

Unter den direktziehenden Nitrobenzolderivaten spielen die Nitroaniline und deren Derivate eine besondere Rolle, da einige dieser Farbstoffe intensive Färbungen von guter Lichtechtheit ausbilden. Ein Nachteil der bekannten direktziehenden Nitroanilin-Farbstoffe ist jedoch einerseits eine begrenzte Wasserlöslichkeit, die zu Problemen bei der Formulierung der Haarfärbemittel führt, andererseits die unbefriedigende Waschechtheit, d. h. daß die Haarfärbungen nach mehrmaligem Haarwaschen stark ausbluten.

Weiterhin ist es wünschenswert, um modische Haarfärbungen zu erhalten, auch rote Farbtöne durch direktziehende Farbstoffe zu erzeugen. Dafür werden gewöhnlich 2-Nitro-p-phenylendiamin und dessen amino-substituierte Derivate verwendet. Leider sind diese Stoffe in Wasser wenig löslich bzw. schwer dispergierbar. Dies führt leicht zu ungleichmäßigen oder zu schwachen Haarfärbungen. Insbesondere bei Färbezubereitungen mit hoher Farbstoffkonzentration kommt es leicht vor, daß Farbstoffe auskristallisieren und nicht auf das zu färbende Haar aufziehen. Es besteht daher ein dringendes Bedürfnis an direktziehenden Haarfarbstoffen mit verbesserter Waserlöslichkeit.

Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich.

Aus EP-A-0 195 361 und US-A-4,494,953 waren Diphenylamin-Derivate bekannt, die sich als Oxidationsfarbstoff-Vorprodukte für die Haarfärbung eignen. Aus EP 0 010 244 war ein Verfahren zur Herstellung von 4-Nitro-4'-amino-diphenylamin-2-sulfonsäure und von 2-Nitro-4'-amino-diphenylamin-4-sulfonsäure bekannt. Diese Verbindungen sollen durch Reduktion in Farbstoffvorprodukte überführt werden.

Es wurde gefunden, daß die gestellten Anforderungen in hohem Maße erfüllt werden durch Haarfärbemittel mit einem Gehalt an direktziehenden Haarfarbstoffen in einem wäßrigen kosmetischen Träger, dadurch gekennzeichnet, daß als direktziehende Haarfarbstoffe eine oder mehrere Verbindungen der Formel (I)

(I)

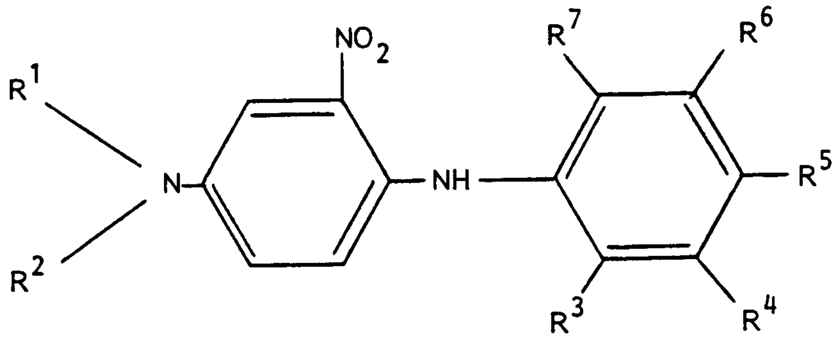

in der $R^1$ und $R^2$ Wasserstoff, Alkylgruppen mit 1 bis 4 C-Atomen oder Hydroxyalkylgruppen mit 2 bis 4 C-Atomen und eine der Gruppen $R^3$ bis $R^7$ eine $-SO_3H$ oder eine -COOH-Gruppe ist und die anderen Wasserstoff, Chlor, Alkylgruppen mit 1 bis 4 C-Atomen, Alkoxygruppen mit 1 bis 4 C-Atomen oder Gruppen der Formel $-NR^8R^9$ sind, worin $R^8$ und $R^9$ Wasserstoff, Alkylgruppen mit 1 bis 4 C-Atomen, Hydroxyalkylgruppen mit 2 bis 4 C-Atomen sind oder gemeinsam mit dem Stickstoffatom einen Piperidin-, Morpholin-, Piperazin- oder Pyrrolidinring bilden, und deren wasserlösliche Salze enthalten sind.

Unter den Verbindungen der Formel (I) sind besonders solche bevorzugt, in welchen eine der Gruppen $R^3$ bis $R^7$ eine COOH-oder eine $-SO_3H$-Gruppe ist und wenigstens zwei der übrigen Wasserstoff sind. Wegen ihrer leichten technischen Zugänglichkeit sind besonders jene Verbindungen bevorzugt, in welchen eine der Gruppen $R^3$ bis $R^7$ eine COOH- oder $-SO_3H$-Gruppe ist und die anderen Wasserstoff sind oder eine der übrigen Chlor, eine Methylgruppe, eine Methoxygruppe oder eine Gruppe $-NR^8R^9$ ist,worin nicht beide $R^8$ und $R^9$ Wasserstoff sind, und die anderen Wasserstoff sind.

Die Farbstoffe der Formel (I) erzeugen auf dem Haar tiefgelbe, braune, rote und blaue Farbnuancen von hoher Intensität, Lichtund Waschechtheit. Gegenüber den Isomeren, in welchen die Stellung der $-NR^1R^2$-Gruppe und der $NO_2$-Gruppe vertauscht ist, zeigen die erfindungsgemäßen Nitrophenylendiamin-Derivate eine deutlich erhöhte Lichtechtheit.

Die Verbindungen der Formel (I) sind neu und daher ein weiterer Gegenstand der Erfindung.

Die Herstellung der Verbindungen der Formel (I) erfolgt allgemein durch Umsetzung von 4-Fluor-3-nitroanilinen der Formel (II)

(II)

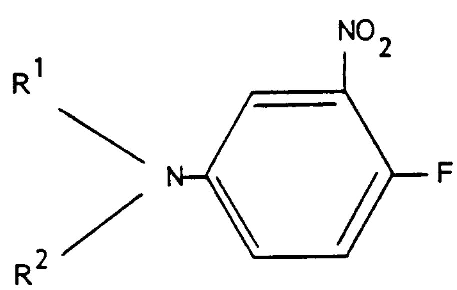

mit einer Aminobenzolsulfonsäure oder Aminobenzoesäure der Formel (III)

(III) 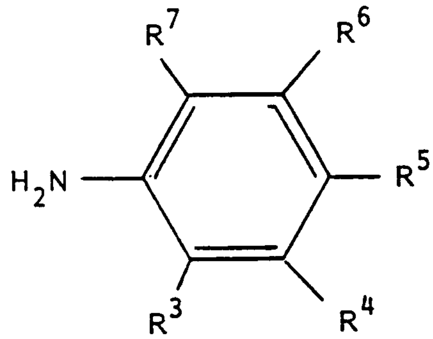

unter Abspaltung von HF in Gegenwart einer Base, z. B. eines Alkalicarbonats, wobei in den Formeln (II) und (III) die Gruppen $R^1$ bis $R^7$ die gleiche Bedeutung haben wie in Formel (I).

Unter den wasserlöslichen Salzen sind in erster Linie die Salze starker Basen wie etwa die Alkalisalze, z. B. das Natrium- oder Kaliumsalz oder Ammoniumsalze, Alkanolammoniumsalze mit 2 bis 4 C-Atomen in der Alkanolgruppe wie z. B. das Monoethanolammoniumsalz, das Triethanolammoniumsalz oder das Isopropanolammoniumsalz zu verstehen. Die erfindungsgemäßen Haarfärbemittel können die direktziehenden Nitrodiphenylaminderivate der allgemeinen Formel I allein oder in Kombination mit bekannten direktziehenden Farbstoffen, z. B. mit anderen Nitrobenzolderivaten, Anthrachinonfarbstoffen, Triphenylmethan- oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifkation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl-oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew,-% des gesamten Färbemittels eingesetzt.

In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 bis 2 Gew.-%, bezogen auf das gesamte Haarfärbemittel, eingesetzt. Daneben können bekannte Oxidationshaarfärbemittelvorprodukte (Entwickler- und Kupplersubstanzen) in einer Menge von 0,01 bis 5, vorzugsweise von 1 bis 3 Gew.-% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, in schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 ° C und 40 ° C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

## Beispiele

### Herstellungsbeispiele

1. 2-Nitro-4-aminodipenylamin-2′-carbonsäure

Eine Mischung aus (A) 27,4 g (0,2 Mol) Anthranilsäure, (B) 31,2 g (0,2 Mol) 4-Fluor-3-nitroanilin, 27,6 g (0,2 Mol) Kaliumcarbonat und ca. 0,1 g Kupfer-Pulver wurde auf ca. 130 ° C erhitzt. Nach 3 Stunden Reaktionszeit bei dieser Temperatur wurde die Mischung abgekühlt und mit 200 ml Wasser ausgekocht. Nach dem Abfiltrieren ungelöster Anteile wurde die heiße (80 ° C) wäßrige Lösung mit verd. Salzsäure auf pH = 3 eingestellt. Der rote Niederschlag wurde abfiltriert und getrocknet.

Rote Kristalle, Schmelzpunkt ca. 212 ° C (unter Zersetzung)

2. 2-Nitro-4-N,N-bis-(2-hydroxyethyl)-aminodiphenylamin-2′-carbonsäure

Die Herstellung erfolgte analog Beispiel 1 ausgehend von
(A) Anthranilsäure und
(B) 4-Fluor-3-nitro-N,N-bis-(2-hydroxyethyl)-aminobenzol
Rote Kristalle, Schmelzpunkt ca. 70 ° C (unter Zersetzung)

3. 2-Nitro-4-amino-4′-chlordiphenylamin-2′-carbonsäure

Herstellung analog Beispiel 1 ausgehend von
(A) 2-Amino-5-chlorbenzoesäure und
(B) 4-Fluor-3-nitroanilin
Dunkelrotes Pulver, Schmelzpunkt 284 ° C (unter Zersetzung)

4. 2-Nitro-4-amino-4′-methyldiphenylamin-2′-carbonsäure

Herstellung analog Beispiel 1 ausgehend von
(A) 2-Amino-5-methyl-benzoesäure und
(B) 4-Fluor-3-nitroanilin
Braunrotes Pulver, Schmelzpunkt 257 ° C bis 263 ° C

5. 2-Nitro-4-amino-5′-chlordiphenylamin-2′-carbonsäure

Herstellung analog Beispiel 1 ausgehend von
(A) 2-Amino-4-chlorbenzoesäure
(B) 4-Fluor-3-nitroanilin
Rotbraunes Pulver, Schmelzpunkt ca. 260 ° C (unter Zersetzung)

6. 2-Nitro-4-N,N-bis-(2-hydroyethyl)-aminodiphenylamin-4′-carbonsäure-Kaliumsalz

Eine Mischung aus (A) 3,4 g (0,025 Mol) 4-Aminobenzoesäure, (B) 6,2 g (0,025 Mol) 4-Fluor-3-nitro-N,N-bis-(2-hydroxyethyl)-aminobenzol, 3,8 g (0,027 Mol) Kaliumcarbonat und 10 ml Wasser wurde in einem

Autoklaven 7 Stunden auf 120 °C erhitzt. Nach dem Abkühlen wurde die Lösung zur Trockene eingeengt. Der Rückstand wurde aus einem Ethanol-Wasser-Gemisch umkristallisiert.

Rote Kristalle, Schmelzpunkt über 250 °C

7. 2-Nitro-4-amino-3′-methyldiphenylamin-2′-carbonsäure

Herstellung analog Beispiel 1 ausgehend von
(A) 2-Amino-6-methylbenzoesäure
(B) 4-Fluor-3-nitroanilin
Dunkelrotes Pulver, Schmelzpunkt ab 164 °C (Zersetzung)

8. 2-Nitro-4-amino-4′-piperidinodiphenylamin-2′-carbonsäure

Herstellung analog Beispiel 1 ausgehend von
(A) 2-Amino-5-piperidinobenzoesäure
(B) 4-Fluor-3-nitroanilin
Schwarze Kristalle, Schmelzpunkt 140 bis 150 °C (Zersetzung)

9. 2-Nitro-4-amino-4′-morpholinodiphenylamin-2′-carbonsäure

Herstellung analog Beispiel 1 ausgehend von
(A) 2-Amino-5-morpholinobenzoesäure
(B) 4-Fluor-3-nitroanilin
Schwarze Kristalle, Schmelzpunkt 186 bis 210 °C (Zersetzung)

10. 2-Nitro-4-amino-4′-methylaminodiphenylamin-2′-carbonsäure

Herstellung analog Beispiel 1 ausgehend von
(A) 2-Amino-5-methylaminobenzoesäure
(B) 4-Fluor-3-mitroanilin

11. 2-Nitro-4-amino-4′,5′-dimethoxydiphenylamin-2′-carbonsäure

Herstellung analog Beispiel 1 ausgehend von
(A) 2-Amino-4,5-dimethoxybenzoesäure
(B) 4-Fluor-3-nitroanilin
Dunkelrote Kristalle, Schmelzpunkt ab 182 °C (Zersetzung)

12. 2-Nitro-4-aminodiphenylamin-3′-sulfonsäure

Herstellung analog Beispiel 1 ausgehend von
(A) 3-Aminobenzolsulfonsäure und
(B) 4-Fluor-3-nitroanilin
Braune Kristalle, Schmelzpunkt ab ca. 285 °C (unter Zersetzung)

13. 2-Nitro-4-dimethylaminodiphenylamin-3′-sulfonsäure-Kaliumsalz

Herstellung analog Beispiel 6 ausgehend von
(A) 3-Aminobenzolsulfonsäure und
(B) N,N-Dimethyl-3-nitro-4-fluoranilin
Violettes Pulver, UV-Spektrum (pH = 9,5); max = 486 nm

14. 2-Nitro-4-amino-4′-methoxydiphenylamin-2′-carbonsäure

Herstellung analog Beispiel 6 ausgehend von
(A) 5-Methoxy-2-aminobenzoesäure und
(B) 4-Fluor-3-nitroanilin
Violette Kristalle, Schmelzpunkt 202 °C (unter Zersetzung)

15. 2-Nitro-4-amino-4′-dimethylaminodiphenylamin-2′-carbonsäure

Herstellung analog Beispiel 1 ausgehend von
(A) 5-Dimethylamino-2-aminobenzoesäure aus 5-Dimethylamino-2-nitrobenzoesäure durch katalytische Hydrierung über Pol (Kohle) in Ethanol bei 25 °C, weißes Pulver, Schmelzpunkt 213 °C und
(B) 4-Fluor-3-nitroanilin
Schwarzes Pulver, Schmelzpunkt 180 bis 190 °C

16. 2-Nitro-4-amino-6'-methoxydiphenylamin-2-carbonsäure

Herstellung analog Beispiel 1 ausgehend von
(A) 2-Amino-3-methoxybenzoesäure und
(B) 4-Fluor-3-nitroanilin
Dunkelrotes Pulver, Schmelzpunkt ca. 141 °C (Zersetzung)

17. 2-Nitro-4-amino-4'-methoxydiphenylamin-2'-carbonsäure

Herstellung analog Beispiel 1 ausgehend von
(A) 2-Amino-5-methoxy-benzoesäure und
(B) 4-Fluor-3-nitroanilin
Rotviolettes Pulver, Schmelzpunkt 202 bis 242 °C (Zersetzung)

18. 2-Nitro-4-amino-4'-(N,N-dimethylamino)-diphenylamin-3'-sulfonsäure

Herstellung analog Beispiel 1 ausgehend von
(A) 2-Dimethylamino-5-aminobenzolsulfonsäure und
(B) 4-Fluor-3-nitroanilin
Dunkelrotes Pulver, Schmelzpunkt über 30 °C

Haarfärbeversuche

Es wurden Haarfärbecremes der folgenden Zusammensetzung hergestellt:

| | |
|---|---|
| Fettalkohol $C_{12-18}$ Fettalkohol $C_{12-14}$ + 2 EO-sulfat, | 10 g |
| Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiel 1 bis 9 eingesetzt.

Das Ergebnis der Färbeversuche ist der Tabelle 1 zu entnehmen.

Tabelle 1

| direktziehender Farbstoff gemäß Beispiel | Nuance des gefärbten Haares |
|---|---|
| 1 | violettbraun |
| 2 | rubin |
| 3 | violettbraun |
| 4 | dunkelmagenta |
| 5 | rotbraun |
| 6 | rubin |
| 7 | braunorange |
| 8 | purpurgrau |
| 9 | graumagenta |
| 10 | mattviolett |
| 11 | violettbraun |
| 12 | violettbraun |
| 13 | tiefgelb |
| 14 | dunkelpurpur |
| 15 | indigo |
| 16 | graubraun |
| 17 | dunkelpurpur |
| 18 | graurot |

**Patentansprüche**

**1.** Haarfärbemittel mit einem Gehalt an direktziehenden Haarfarbstoffen, dadurch gekennzeichnet, daß als direktziehende Haarfarbstoffe eine oder mehrere Verbindungen der Formel (I)

( I )

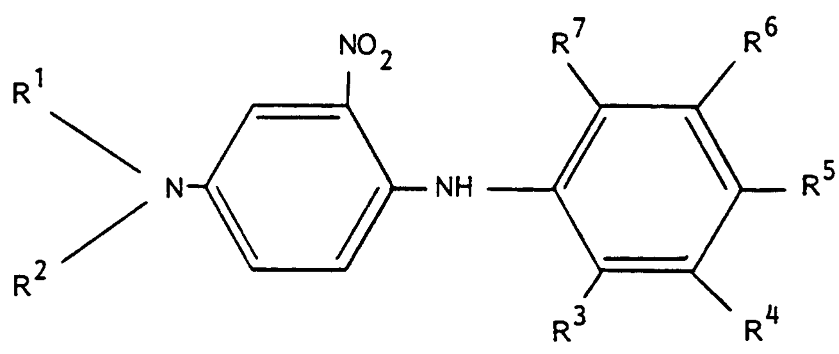

in der $R^1$ und $R^2$ Wasserstoff, Alkylgruppen mit 1 bis 4 C-Atomen oder Hydroxyalkylgruppen mit 2 bis 4 C-Atomen und eine der Gruppen $R^3$ bis $R^7$ eine $-SO_3H$ oder eine -COOH-Gruppe ist und die anderen Wasserstoff, Chlor, Alkylgruppen mit 1 bis 4 C-Atomen, Alkoxygruppen mit 1 bis 4 C-Atomen oder Gruppen der Formel $-NR^8R^9$ sind, worin $R^8$ und $R^9$ Wasserstoff, Alkylgruppen mit 1 bis 4 C-Atomen, Hydroxyalkylgruppen mit 2 bis 4 C-Atomen sind oder gemeinsam mit dem Stickstoffatom einen Piperidin-, Morpholin-, Piperazin- oder Pyrrolidinring bilden, und deren wasserlösliche Salze enthalten sind.

**2.** Haarfärbemittel nach Anspruch 1, dadurch gekennzeichnet, daß eine der Gruppen $R^3$ bis $R^7$ eine COOH- oder eine $-SO_3H$-Gruppe ist und wenigstens zwei der übrigen Wasserstoff sind.

**3.** Haarfärbemittel nach den Ansprüchen 1 oder 2, dadurch gekennzeichnet, daß die direktziehenden Haarfarbstoffe der Formel (1) in einer Menge von 0,01 bis 5 Gew.-%, bezogen auf das gesamte

Haarfärbemittel, enthalten sind.

**4.** Haarfärbemittel nach den Ansprüchen 1 bis 3, dadurch gekennzeichnet, daß zusätzlich zu den Verbindungen der Formel (I) noch weitere direktziehende Haarfarbstoffe und/oder Oxidationsfarbstoffvorprodukte enthalten sind und daß diese Farbstoffe in einem kosmetischen Träger in Form einer Creme, einer Emulsion, eines Gels, eines Shampoos oder eines Schaumaerosols eingearbeitet sind.

**5.** Nitrodiphenylaminderivate der Formel (I)

( I ) 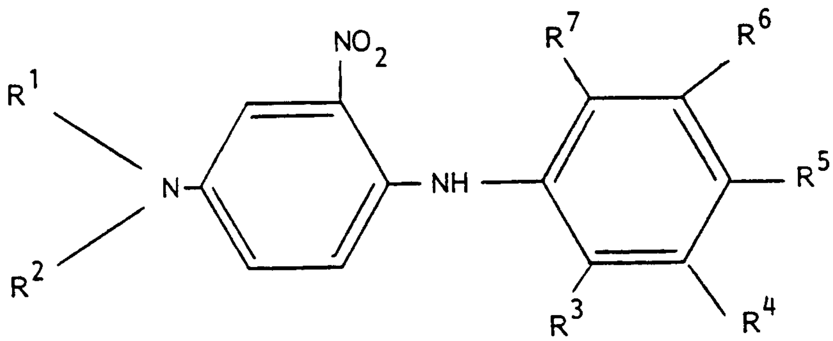

in der $R^1$ und $R^2$ Wasserstoff, Alkylgruppen mit 1 bis 4 C-Atomen oder Hydroxyalkylgruppen mit 2 bis 4 C-Atomen und eine der Gruppen $R^3$ bis $R^7$ eine $SO_3H$- oder eine -COOH-Gruppe ist und die übrigen Wasserstoff, Chlor, Alkylgruppen mit 1 bis 4 C-Atomen, Alkoxygruppen mit 1 bis 4 C-Atomen oder Gruppen der Formel $-NR^8R^9$ sind, worin $R^8$ und $R^9$ Wasserstoff, Alkylgruppen mit 1 bis 4 C-Atomen, Hydroxyalklygruppen mit 2 bis 4 C-Atomen sind oder gemeinsam mit dem Stickstoffatom einen Piperidin-, Morpholin-, Piperazin-oder Pyrrolidinring bilden, und deren wasserlösliche Salze.

**Claims**

**1.** Hair-dyeing preparations containing substantive hair dyes, characterized in that one or more compounds corresponding to the following formula

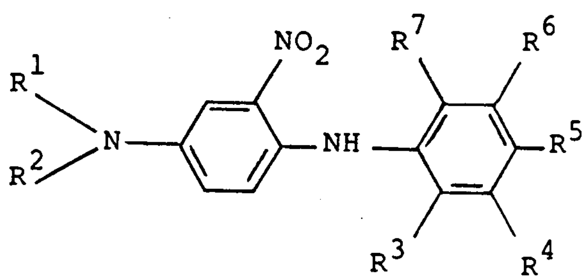 (I)

in which $R^1$ and $R^2$ are hydrogen, $C_1$-$C_4$ alkyl groups or $C_2$-$C_4$ hydroxyalkyl groups and one of the substituents $R^3$ to $R^7$ is an $-SO_3H$ or -COOH group and the other substituents are hydrogen, chlorine, $C_1$-$C_4$ alkyl groups, $C_1$-$C_4$ alkoxy groups or groups of the formula $-NR^8R^9$, where $R^8$ and $R^9$ are hydrogen, $C_1$-$C_4$ alkyl groups, $C_2$-$C_4$ hydroxyalkyl groups or, together with the nitrogen atom, form a piperidine, morpholine, piperazine or pyrrolidine ring,
and water-soluble salts thereof are present as the substantive hair dyes.

**2.** Hair-dyeing preparations as claimed in claim 1, characterized in that one of the substituents $R^3$ to $R^7$ is a -COOH group or an $-SO_3H$ group and at least two of the other substituents are hydrogen.

**3.** Hair dyeing preparations as claimed in claim 1 or 2, characterized in that the substantive dyes corresponding to formula (I) are present in a quantity of from 0.01 to 5% by weight, based on the hair-dyeing preparation as a whole.

**4.** Hair-dyeing preparations as claimed in claims 1 to 3, characterized in that other substantive hair dyes and/or oxidation hair dye precursors are present in addition to the compounds corresponding to formula (I) and in that these dyes are incorporated in a cosmetic carrier in the form of a cream, an emulsion, a gel, a shampoo or a foam aerosol.

**5.** Nitrodiphenylamine derivatives corresponding to the following formula

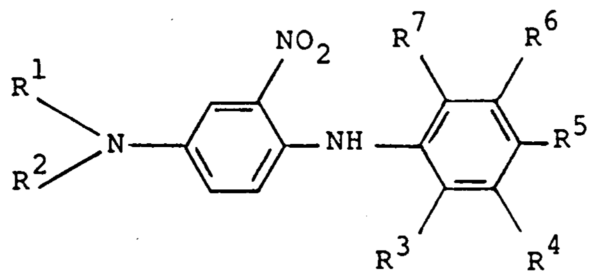

(I)

in which $R^1$ and $R^2$ are hydrogen, $C_1$-$C_4$ alkyl groups or $C_2$-$C_4$ hydroxyalkyl groups and one of the substituents $R^3$ to $R^7$ is an $-SO_3H$ or -COOH group and the other substituents are hydrogen, chlorine, $C_1$-$C_4$ alkyl groups, $C_1$-$C_4$ alkoxy groups or groups of the formula $-NR^8R^9$, where $R^8$ and $R^9$ are hydrogen, $C_1$-$C_4$ alkyl groups, $C_2$-$C_4$ hydroxyalkyl groups or, together with the nitrogen atom, form a piperidine, morpholine, piperazine or pyrrolidine ring,
and water-soluble salts thereof.

## Revendications

**1.** Teinture pour cheveux ayant une teneur en colorants capillaires à teinte directe, caractérisée en ce que comme colorant capillaire à teinte directe, un ou plusieurs composés de formule (I) :

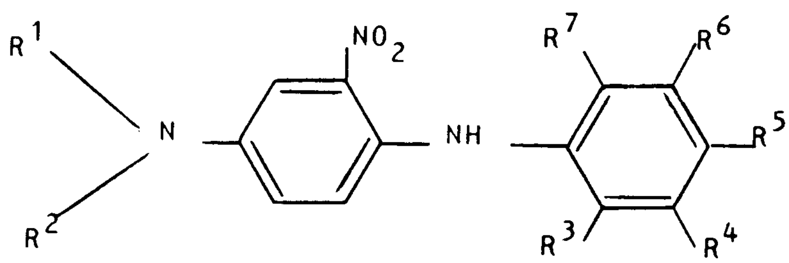

(I)

dans laquelle $R_1$ et $R_2$ sont de l'hydrogène, des radicaux alcoyles ayant de 1 à 4 atomes de carbone ou des groupes hydroxyalcoyles ayant de 2 à 4 atomes de carbone et un des groupes $R_3$ à $R_7$ représente un groupe $-SO_3H$ ou -COOH et les autres groupes représentent de l'hydrogène, du chlore, des radicaux alcoyles ayant de 1 à 4 atomes de carbone, des groupes alcoxy ayant de 1 à 4 atomes de carbone ou des groupes de formule

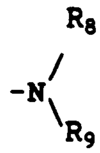

dans laquelle $R_8$ et $R_9$ sont de l'hydrogène, des radicaux alcoyles ayant de 1 à 4 atomes de carbone, des radicaux hydroxyalcoyles ayant de 2 à 4 atomes de carbone ou ensemble avec l'atome d'azote forment un cycle pipéridinique, morpholinique, pipérazine, ou pyrolidine et leurs sels solubles dans l'eau sont contenus.

**2.** Teinture pour cheveux selon la revendication 1, caractérisée en ce qu'un des groupes $R_3$ à $R_7$ est un groupe-COOH ou $-SO_3H$ et au moins deux des autres sont de l'hydrogène.

**3.** Teinture pour cheveux selon les revendications 1 ou 2, caractérisée en ce que les colorants à teinte directe de formule (I) sont contenus en quantité de 0,01 à 5 % en poids rapporté à la teinture pour cheveux totale.

**4.** Teinture pour cheveux selon les revendications 1 à 3, caractérisée en ce qu'on addition aux composés de formule (I), d'autres colorants supplémentaires à teinte directe et/ou des précurseurs de colorants par oxydation sont contenus et en ce que ces colorants sont incorporés dans un support cosmétique sous la forme d'une crème, d'une émulsion, d'un gel, d'un shampooing ou d'un aérosol en mousse.

**5.** Dérivés de la nitrodiphénylamine de formule (I) :

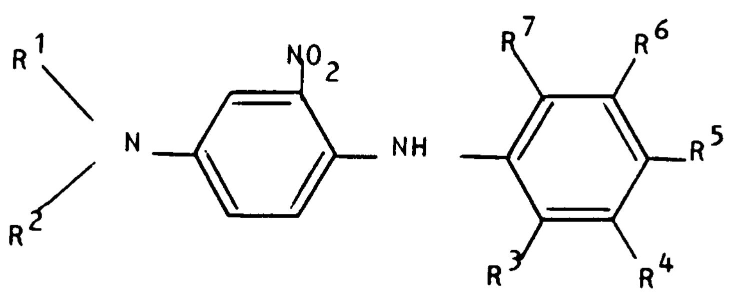

(I)

dans laquelle $R_1$ et $R_2$ sont de l'hydrogène, des radicaux alcoyle ayant de 1 à 4 atomes de carbone ou des groupes hydroxyalcoyles ayant de 2 à 4 atomes de carbone et un des groupes $R_3$ à $R_7$ est un groupe $SO_3H$-ou -COOH et les autres substituants sont de l'hydrogène, du chlore, des radicaux alcoyles ayant de 1 à 4 atomes de carbone, des radicaux alcoxy ayant de 1 à 4 atomes de carbone ou sont des groupes de formule

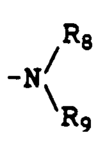

dans laquelle $R_8$ t $R_9$ représentent de l'hydrogène, des radicaux alcoyles ayant de 1 à 4 atomes de carbone, des groupes hydroxyalcoyle ayant de 2 à 4 atomes de carbone, ou forment ensemble avec l'atome d'azote un cycle pipéridine, morpholine, pipérazine ou pyrolidine ainsi que leurs sels solubles dans l'eau.